(19) Europäisches Patentamt
European Patent Office
Office européen des brevets

(11) Veröffentlichungsnummer: **0 038 987**
**B1**

(12) # EUROPÄISCHE PATENTSCHRIFT

(45) Veröffentlichungstag der Patentschrift: **08.06.83**

(51) Int. Cl.³: **C 07 C 120/04,** C 07 C 121/76, C 07 C 121/34 // C07D253/06

(21) Anmeldenummer: **81102795.2**

(22) Anmeldetag: **11.04.81**

(54) **Verfahren zur Herstellung von Acylcyaniden.**

(30) Priorität: **23.04.80 DE 3015587**

(43) Veröffentlichungstag der Anmeldung: **04.11.81 Patentblatt 81/44**

(45) Bekanntmachung des Hinweises auf die Patenterteilung: **08.06.83 Patentblatt 83/23**

(84) Benannte Vertragsstaaten: **AT BE CH DE FR GB IT LI NL**

(56) Entgegenhaltungen:
**DE-A-2 614 240**
**DE-A-2 614 242**
**DE-A-2 642 140**
**DE-A-2 708 183**

**HOUBEN-WEYL: «Methoden der organischen Chemie», 4. Auflage, Band V/3, 1962, GEORG THIEME VERLAG, Stuttgart, Seiten 419, 420***

(73) Patentinhaber: **BAYER AG, Zentralbereich Patente, Marken und Lizenzen, D-5090 Leverkusen 1, Bayerwerk (DE)**

(72) Erfinder: **Klauke, Erich, Dr., Eichendorffweg 8, D-5068 Odenthal (DE)**
Erfinder: **Findeisen, Kurt, Dr., In der Follmühle 10, D-5068 Odenthal (DE)**

**EP 0 038 987 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von Acylcyaniden

Die vorliegende Erfindung betrifft ein Verfahren zur Herstellung von teilweise bekannten Acylcyaniden, welche als Ausgangsstoffe zur Synthese von Herbiziden verwendet werden können.

Nach einem früheren Vorschlag der Anmelderin kann man Acylcyanide dadurch erhalten, dass man Carbonsäurehalogenide mit Alkalicyaniden in Gegenwart katalytischer Mengen eines Schwermetallcyanids, z.B. von Kupfercyanid, und gegebenenfalls in Gegenwart eines Verdünnungsmittels, bei Temperaturen zwischen 100°C und 300°C umsetzt (vgl. DE-OS 2 614 242). Nachteilig bei diesem Verfahren ist jedoch die Notwendigkeit, dass Schwermetallcyanide als Katalysator eingesetzt werden müssen (zum älteren Stand der Technik vgl. J. Thesing et al., Angew. Chem. 68, S. 425-435 [1956]).

Es wurde nun überraschend gefunden, dass man die teilweise bekannten Acylcyanide der allgemeinen Formel

R-CO-CN (I),

in welcher

R für gegebenenfalls substituiertes Alkyl mit bis zu 8 Kohlenstoffatomen, gegebenenfalls substituiertes Cycloalkyl mit 3 bis 12 Kohlenstoffatomen, gegebenenfalls substituiertes Aryl oder für einen gegebenenfalls substituierten 5- oder 6-gliedrigen heterocyclischen Rest steht, der zusätzlich noch mit einem Benzolring anelliert sein kann,

durch Umsetzung von Carbonsäurefluoriden der allgemeinen Formel

R-CO-F (II),

in welcher R die oben angegebene Bedeutung hat, mit Alkalicyaniden, gegebenenfalls in Gegenwart eines Verdünnungsmittels, in hohen Ausbeuten und hoher Reinheit erhält, wenn man die Umsetzung in Abwesenheit eines Katalysators, bei Temperaturen zwischen 10 und 200°C, durchführt.

Es ist als ausgesprochen überraschend zu bezeichnen, dass Acylcyanide der Formel (I) nach dem erfindungsgemässen Verfahren in hoher Ausbeute und ausgezeichneter Reinheit zugänglich sind. Dabei ist es besonders überraschend, dass die Carbonsäurefluoride unter milderen Bedingungen als die entsprechenden Carbonsäurechloride, nämlich ohne Schwermetallcyanid als Katalysator und bei relativ niederen Temperaturen, mit Alkalicyaniden reagieren.

Bekannt ist, dass die gewöhnlichen Carbonsäurefluoride durchweg weniger reaktionsfähig sind als die anderen Carbonsäurehalogenide, eingeschlossen Carbonsäurechloride (vgl. Houben-Weyl, Methoden der Organischen Chemie, 4. Auflage, Band V/3, S. 419 [1962]). Im Hinblick auf den bekannten Stand der Technik war daher zu erwarten, dass sich Carbonsäurefluoride nur unter schärferen Reaktionsbedingungen im gleichen Sinne wie die Carbonsäurechloride umsetzen lassen bzw. dass ohne Zusatz eines Schwermetallcyanids als Katalysator praktisch keine Umsetzung der Carbonsäurefluoride mit den Alkalicyaniden eintreten würde.

Das erfindungsgemässe Verfahren besitzt eine Reihe von Vorteilen. Ein wichtiger Vorteil besteht darin, dass bei der Herstellung keine Kupfersalze oder anderen Schwermetallsalze eingesetzt werden müssen. Weiterhin ist es von Vorteil, dass das erfindungsgemässe Verfahren unter milderen Bedingungen abläuft als das aus DE-OS 2 614 242 vorbekannte Verfahren; während bei dem vorbekannten Verfahren die optimalen Reaktionstemperaturen durchweg bei etwa 200°C oder höher liegen, liegen diese bei dem erfindungsgemässen Verfahren im Bereich von etwa 100°C oder niedriger, wie die jeweiligen präparativen Herstellungsbeispiele zeigen. Des weiteren ist das erfindungsgemässe Verfahren nicht auf die Synthese bestimmter weniger Verbindungen beschränkt, sondern es lässt sich sehr bereit anwenden. Ganz abgesehen davon ist es auch in technischem Massstab in relativ einfacher Weise durchführbar. Die Acylcyanide fallen bei dem erfindungsgemässen Verfahren - wie bereits erwähnt - in hoher Ausbeute und ausgezeichneter Reinheit frei von störenden Nebenprodukten an.

Verwendet man Benzoylfluorid und Natriumcyanid als Ausgangsstoffe, so kann der Reaktionsverlauf durch das folgende Formelschema wiedergegeben werden:

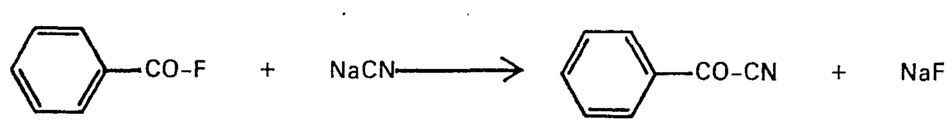

Die als Ausgangsstoffe verwendeten Säurefluoride sind durch die Formel (II) allgemein definiert. In dieser Formel steht R vorzugsweise für Alkyl mit 4 bis 6 Kohlenstoffatomen, wobei jeder dieser Alkylreste substituiert sein kann durch Alkoxy mit 1 bis 4 Kohlenstoffatomen, Carbalkoxy mit 1 bis 4 Kohlenstoffatomen in der Alkoxygruppe, Nitro, Nitril und/oder Halogen, wie z.B. Fluor, Chlor, Brom oder Jod. Ferner steht R vorzugsweise für gegebenenfalls durch Alkyl, Alkoxy oder Carbalkoxy mit jeweils bis zu 4 Kohlenstoffatomen, Nitro, Nitril und/oder Halogen, wie z.B. Fluor, Chlor oder Brom, substituiertes Cycloalkyl mit 3 bis 6 Kohlenstoffatomen im Ringsystem.

Weiterhin steht R vorzugsweise für gegebenenfalls durch Alkyl, Alkoxy, Halogenalkyl, Halogenalkoxy oder Carbalkoxy mit jeweils bis zu 4 Kohlenstoffatomen, Nitro und/oder Halogen, wie z.B. Fluor, Chlor oder Brom, substituiertes Aryl, insbesondere Phenyl oder Naphthyl. Schliesslich steht R vorzugsweise für gegebenenfalls durch Alkyl, Alkoxy oder Carbalkoxy mit jeweils bis zu 4 Kohlenstoffatomen, Nitro, Nitril und/oder Halogen, wie z.B. Fluor, Chlor und Brom, substituierte 5- oder 6-gliedrige heterocyclische Reste, die 1 bis 3 Heteroatome wie Sauerstoff, Schwefel und/oder Stickstoff im Ring enthalten können und ausserdem mit einem Benzolring anelliert sein können. Als Beispiel für insbesondere in Frage kommende heterocyclische Reste seien genannt: Morpholinyl, Imidazolyl, Pyrazolyl, Pyrrolyl, Isoxazolyl, Piperidinyl, Oxazolyl, 1,2,4-Triazol-1-yl, 1,2,4-Triazol-4-yl, 1,2,3-Triazolyl, 1,2,4-Thiadiazol-2-yl, Benzimidazolyl und Furanyl.

Die als Ausgangsstoffe zu verwendenden Carbonsäurefluoride der Formel (II) sind bekannt oder lassen sich nach prinzipiell bekannten Methoden herstellen, z.B. durch Umsetzung der entsprechenden Carbonsäurechloride mit Fluorwasserstoff oder Alkalifluoriden oder Alkalihydrogenfluoriden (vgl. Houben-Weyl, Methoden der Organischen Chemie, 4. Aufl., Bd. V/3, S. 119–120 und 148–150 [1962] sowie Herstellungsbeispiele).

Als bevorzugte Beispiele für Carbonsäurefluoride seien im einzelnen genannt:

Pivalinsäurefluorid, Hexancarbonsäurefluorid, Cyclopropancarbonsäurefluorid, (2,2-Dichlor-1-methylcyclopropyl)-carbonsäurefluorid, (2,2-Dichlor-1,3-dimethylcyclopropyl)-carbonsäurefluorid, Cyclopentancarbonsäurefluorid, Cyclohexancarbonsäurefluorid, Benzoylfluorid, 3-Chlor-benzoylfluorid,4-Chlor-benzoylfluorid, 2,5-Dichlor-benzoylfluorid, 2-Fluorbenzoylfluorid, 2,6-Difluorbenzoylfluorid, 3-Trifluormethyl-benzoylfluorid, 4-Trifluormethyl-benzoylfluorid, 3-Chlor-4-trifluormethoxy-benzoylfluorid, 3-Brom-4-fluor-benzoylfluorid, 4-Nitrobenzoyl-fluorid, 3,5-Dinitrobenzoylfluorid, 3-Nitro-4-methyl-benzoylfluorid, 4-Methyl-benzoylfluorid, Naphthalin-1-carbonsäurefluorid, 1-Phenyl-5-pyrazolon-3-carbonsäurefluorid, sowie die weiteren in den Beispielen aufgeführten Carbonsäurefluoride.

Selbstverständlich ist es auch möglich, an Stelle der Monocarbonsäurefluoride (II) Dicarbonsäure-difluoride – in entsprechender Menge – bei dem erfindungsgemässen Verfahren einzusetzen. Als Beispiele hierfür seinen Terephthalsäuredifluorid und Isophthalsäuredifluorid angeführt.

Als besonders bevorzugte Carbonsäurefluoride (II) seien Benzoylfluorid, 3-Trifluormethyl-benzoylfluorid und Pivalinsäurefluorid genannt.

Als Alkalicyanide kommen insbesondere Natrium- und Kaliumcyanid in Frage. Bevorzugt wird Natriumcyanid verwendet.

Die erfindungsgemässe Umsetzung kann in Anwesenheit oder in Abwesenheit eines Verdünnungsmittels durchgeführt werden.

Als Verdünnungsmittel kommen alle inerten organischen Lösungsmittel, die weder mit den Carbonsäurefluoriden noch mit den Alkalicyaniden eine chemische Reaktion eingehen, in Betracht. Als solche Lösungsmittel seien genannt: Methylenchlorid, Chloroform, Essigester, Glykoldimethyläther, Acetonitril, Sulfolan, Toluol, Xylol, Chlorbenzol, Dichlorbenzol.

Ausserdem ist es möglich, im Überschuss eingesetztes Carbonsäurefluorid (II) als Verdünnungsmittel zu verwenden.

Die Reaktionstemperaturen können in einem grösseren Bereich variiert werden. Im allgemeinen arbeitet man – wie oben angegeben – bei Temperaturen zwischen 10 und 200°C, beim Arbeiten in Gegenwart eines Verdünnungsmittels vorzugsweise zwischen 20 und 150°C, beim Arbeiten ohne Verdünnungsmittel vorzugsweise zwischen 60 und 120°C, besonders bevorzugt zwischen 80 und 100°C.

Das erfindungsgemässe Verfahren wird im allgemeinen unter Normaldruck durchgeführt. Bei der Umsetzung niedrigsiedender Carbonsäurefluoride empfiehlt sich jedoch zur Erhöhung des Umsatzes die Anwendung von Überdruck, im allgemeinen bis 15 bar.

Bei der Durchführung des erfindungsgemässen Verfahrens setzt man auf 1 Mol Carbonsäurefluorid (II) im allgemeinen 0,7 bis 1,4 Mol Alkalicyanid, vorzugsweise 0,8 bis 1,3 Mol Alkalicyanid ein. Besonders bevorzugt ist ein Molverhältnis von Carbonsäurefluorid (II) zu Alkalicyanid von 1:1–1,25.

Die Aufarbeitung des Reaktionsgemisches erfolgt nach beendeter Umsetzung in üblicher Weise. Zunächst werden die im Reaktionsverlauf gebildeten Alkalimetallfluoride durch Filtration abgetrennt; sie können zur Herstellung der Carbonsäurefluoride (II) wiederverwendet werden. Das Filtrat wird einer einfachen franktionierten Destillation unterworden, wenn flüssige Acylcyanide (I) isoliert werden sollen.

Feste Acylcyanide (I) erhält man am zweckmässigsten durch Kristallisation aus der gegebenenfalls heiss filtrierten und gegebenenfalls durch Abdestillieren von Verdünnungsmittel eingeengten Reaktionslösung; auf eine weitere Reinigung durch Umkristallisieren kann im Normalfall verzichtet werden.

In einer besonderen Verfahrensvariante lässt sich die erfindungsgemässe Umsetzung auch kontinuierlich gestalten.

Die nach dem erfindungsgemässen Verfahren herstellbaren Acylcyanide der Formel (I) sind wertvolle Ausgangsstoffe z.B. zur Synthese von 1,2,4-Triazin-5-onen, welche hervorragende herbizide Eigenschaften besitzen (vgl. z.B. DE-OS 2 224 161; DE-PS 1 795 784).

So lässt sich beispielsweise das 3-Methyl-4-amino-6-phenyl-1,2,4-triazin-5(4H)-on der Formel

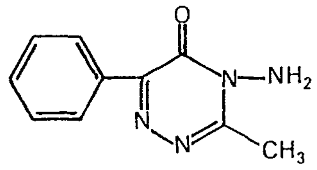

herstellen, indem man in einer ersten Stufe Benzoylcyanid in Gegenwart von konzentrierter Salzsäure mit Äthanol umsetzt und den dabei entstehenden Phenylglyoxylsäureethylester in einer zweiten Stufe mit Acetylhydrazin zur Reaktion bringt, wobei sich 1-Phenyl-glyoxylsäureethylester-2-acetylhydrazon bildet, das in einer dritten Stufe mit Hydrazinhydrat in Gegenwart von Pyridin in das oben erwähnte Endprodukt überführt wird.

Diese mehrstufige Synthese lässt sich formelmässig wie folgt wiedergeben:

1. Stufe:

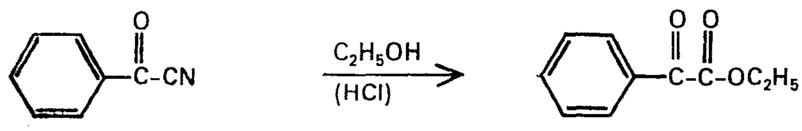


2. Stufe:

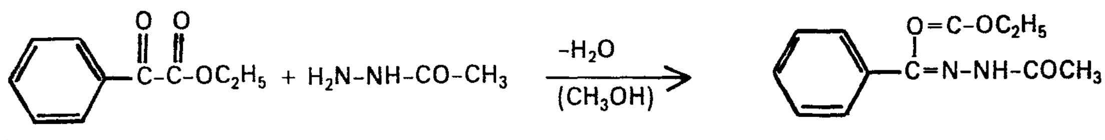


3. Stufe:

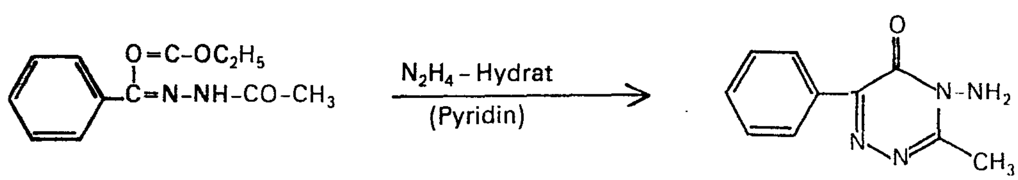


Das erfindungsgemäss herstellbare Pivaloylcyanid lässt sich nach bekannten Verfahren z.B. in den herbiziden Wirkstoff 3-Methylthio-4-amino-6-tert.-butyl-1,2,4-triazin-5-(4H)-on überführen (vgl. DE-OS 2 733 180, US-PS 4 175 188, ferner Deutsche Patentanmeldungen P 30 02 203.8, P 30 03 541.7, P 30 09 043.8).

Das erfindungsgemässe Verfahren wird durch die nachfolgenden Herstellungsbeispiele veranschaulicht:

Herstellungsbeispiele

Beispiel 1

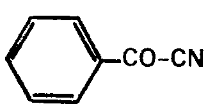

In einem 250-ml-Dreihalskolben werden 62 g Benzoylfluorid (0,5 Mol) und 25 g Natriumcyanid (0,5 Mol) zu 100 ml Acetonitril gegeben. Die Mischung wird gerührt, die Innentemperatur steigt auf 55°C an. Nach dem Abklingen der exothermen Reaktion wird drei Stunden unter Rückfluss gekocht. Anschliessend wird fraktioniert destilliert.

Ausbeute: 63,5 g (97% der Theorie) Benzoylcyanid;

Schmelzpunkt: 33°C.

Beispiel 2

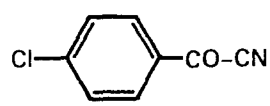

In einem 250-ml-Dreihalskolben mit Rührer, Thermometer und Rückenflusskühler werden 79,3 g 4-Chlor-benzoylfluorid (0,5 Mol) in 100 ml Toluol gegeben und dann mit 25 g Natriumcyanid (0,5 Mol) versetzt. Unter Rühren steigt die Innentemperatur auf 50°C an, anschliessend wird 2 Stunden auf 110°C erwärmt. Das Reaktionsgemisch wird durch Filtration vom Natriumfluorid befreit und anschliessend destilliert.

Ausbeute: 81 g (98% der Theorie) 4-Chlor-benzoylcyanid;

Siedepunkt: 117–119°C bei 16 mbar.

Beispiel 3

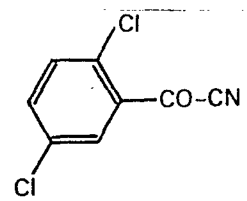

In einem 250-ml-Dreihalskolben werden 92,5 g 2,5-Dichlor-benzoylfluorid (0,5 Mol), 25 g Natriumcyanid (0,5 Mol) und 100 ml Xylol unter Rühren zusammengegeben. Nach dem Abklingen der

exothermen Reaktion wird 1 Stunde unter Rückfluss erwärmt. Das entstandene Natriumfluorid wird abfiltriert, das Filtrat vom Lösungsmittel befreit und der Rückstand destilliert.

Ausbeute: 92 g (92% der Theorie) 2,5-Dichlorbenzoylcyanid;

Siedepunkt: 142–145°C bei 19,5 mbar.

Beispiel 4

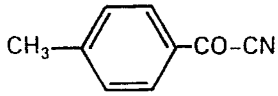

In einem 250-ml-Dreihalskolben werden 68,5 g p-Methylbenzoylfluorid (0,5 Mol) und 25 g Natriumcyanid (0,5 Mol) in 100 ml Methylenchlorid gegeben. Nach beendeter exothermer Reaktion wird 5 Stunden auf Siedetemperatur erwärmt, anschliessend filtriert und destilliert.

Ausbeute: 68 g (94% der Theorie) p-Methylbenzoylcyanid;

Siedepunkt: 114–117°C bei 16 mbar;

Schmelzpunkt: 46–47°C.

Beispiel 5

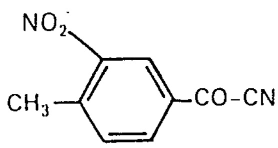

In einem 250-ml-Dreihalskolben werden 78,5 g 3-Nitro-4-methyl-benzoylfluorid (0,5 Mol), 25 g Natriumcyanid (0,5 Mol) und 100 ml Chlorbenzol zusammengegeben. Anschliessend wird drei Stunden unter Rückfluss gekocht. Die Lösung wird anschliessend filtriert, eingeengt und der Rückstand wird aus Isobutanol umkristallisiert.

Ausbeute: 83 g (87% der Theorie) 3-Nitro-4-methyl-benzoylcyanid;

Schmelzpunkt: 140–142°C.

Beispiel 6

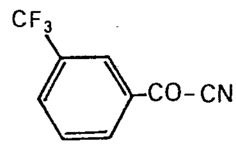

100 g (0,52 Mol) 3-Trifluormethyl-benzoylfluorid werden vorgelegt und 31 g (0,63 Mol) Natriumcyanid werden eingetragen. Die einsetzende Reaktion ist leicht exotherm und das Reaktionsgemisch verfärbt sich nach gelb. Nun heizt man das Reaktionsgemisch innerhalb 30 Minuten bis auf 90°C und rührt noch 2 Stunden bei dieser Temperatur nach. Die Änderung des Brechungsindex im Verlauf der Reaktion ist ein gutes Mass für den Umsatz; er ändert sich von $n_D^{20}$: 1,4390 nach 1,4745. Zur Aufarbeitung wird abgekühlt, filtriert und das Filtrat destilliert.

Man erhält 93 g (90% der Theorie) 3-Trifluormethyl-benzoylcyanid als wasserhelle Flüssigkeit vom Siedepunkt: 93°C/19 mbar, mit dem Brechungsindex $n_D^{20}$: 1,4779.

Unter den gleichen Reaktionsbedingungen lassen sich, ausgehend von den entsprechenden Carbonsäurefluoriden (II), die in der folgenden Tabelle zusammengestellten Acylcyanide (I) herstellen; physikalische Daten sind dabei jeweils für Ausgangs- und Endprodukte angegeben:

**Tabelle**

$$R{-}CO{-}F \longrightarrow R{-}CO{-}CN$$

(II) (I)

| Beispiel Nr. | R | Siedepunkt/Druck<br>Brechungsindex, $n_D^{20}$<br>gegebenenfalls<br>Schmelzpunkt (Fp.) | |
|---|---|---|---|
| | | (II) | (I) |
| 7 | 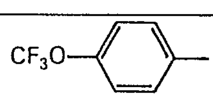 | 94–95°C/100 mbar<br>1,4315 | 99°C/23 mbar<br>1,4775 |
| 8 | 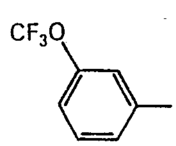 | 53°C/14,6 mbar<br>1,4270 | 94°C/20 mbar<br>1,4688 |
| 9 | 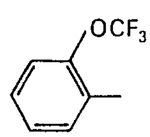 | 60°C/14,6 mbar<br>1,4285 | 104°C/19 mbar<br>1,4742 |

Tabelle (Fortsetzung)

| Beispiel Nr. | R | Siedepunkt/Druck Brechungsindex, $n_D^{20}$ gegebenenfalls Schmelzpunkt (Fp.) (II) | (I) |
|---|---|---|---|
| 10 | 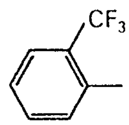 | 70°C/22 mbar 1,4429 | 109°C/20 mbar Fp. 58–59°C |
| 11 | 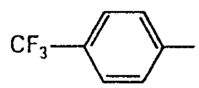 | 159°C/1 bar 1,4399 | 92°C/20 mbar 1,4772 |
| 12 | 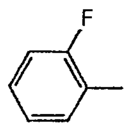 | 171°C/1 bar 1,4865 | 111°C/22 mbar 1,5333 |
| 13 | 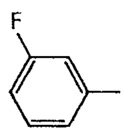 | 40°C/20 mbar 1,4764 | 88°C/20 mbar 1,5272 |
| 14 | 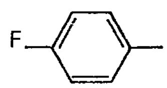 | 52°C/18,6 mbar 1,4792 | 93°C/20 mbar Fp. 23–24°C |
| 15 | 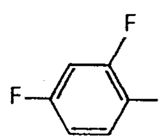 | 53°C/17,3 mbar 1,4680 | 101°C/21 mbar 1,5193 |
| 16 | 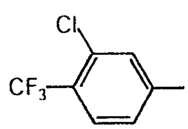 | 75°C/14,6 mbar 1,4659 | 115°C/20 mbar 1,5030 |
| 17 | 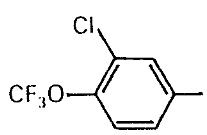 | 78°C/18,6 mbar 1,4545 | 113°C/20 mbar 1,4941 |
| 18 | 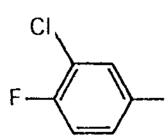 | 72°C/16 mbar 1,5070 | 114°C/18 mbar 1,5539 |
| 19 | 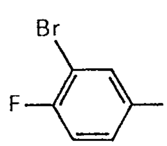 | 83°C/15 mbar Fp. 32–40°C | 126°C/20 mbar 1,5769 |
| 20 | 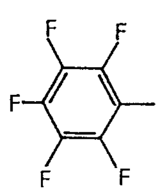 | 140°C/1 bar 1,4270 | 90°C/19 mbar 1,4700 |

Beispiel 21

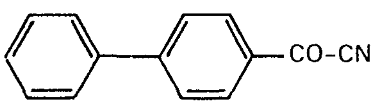

In einem 250-ml-Dreihalskolben werden 20 g p-Diphenylcarbonsäurefluorid (0,1 Mol) in 60 ml Toluol gelöst und mit 5,5 g Natriumcyanid (0,11 Mol) versetzt. Die Reaktionsmischung wird drei Stunden unter Rückfluss gekocht und heiss filtriert. Nach dem Erkalten kristallisiert das p-Diphenylcarbonsäurecyanid aus der Toluollösung aus.

Ausbeute: 19,7 g (96% der Theorie) p-Diphenylcarbonsäurecyanid;

Schmelzpunkt: 129°C.

Beispiel 22

Wie zuvor beschrieben, wird eine Lösung von 20,8 g Pivaloylfluorid (0,2 Mol) in 50 ml Benzonitril mit 11 g Natriumcyanid (0,22 Mol) versetzt. Es tritt eine exotherme Reaktion auf. Danach wird die Innentemperatur auf 130°C gesteigert und es wird 30 Minuten bei dieser Temperatur gerührt. Nach dem Erkalten wird vom Natriumfluorid abfiltriert; der Rückstand wird erst bei Normaldruck, dann im Vakuum destilliert.

Ausbeute: 20,4 g (92% der Theorie) Pivaloylcyanid;

Siedepunkt: 47–52°C bei 20 mbar.

Herstellungsbeispiele für Carbonsäurefluoride (II):

Beispiel a

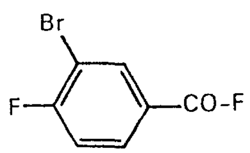

Es werden 1270 g (5 Mol) 4-Chlor-3-brom-benzoylchlorid mit 1150 g Tetramethylensulfon und 315 g (7,5 Mol) wasserfreiem Natriumfluorid unter Rühren 5 Stunden auf 210°C erwärmt. Danach wird das Reaktionsgemisch mit 435 g (7,5 Mol) wasserfreiem Kaliumfluorid, das in 1150 g Tetramethylensulfon suspendiert ist, versetzt und weitere 7 Stunden bei 210°C gerührt. Die anschliessende Destillation liefert 873 g (79% der Theorie) 4-Fluor-3-brom-benzoylfluorid (vgl. Beispiel 19).

Beispiel b

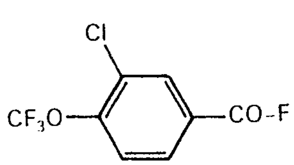

230 g p-Anissäure und 230 g Thionylchlorid werden unter Stickstoff allmählich auf 60°C erwärmt. Das Reaktionsgemisch wird 10 Stunden lang bei 60°C gerührt. Danach wird überschüssiges Thionylchlorid im Vakuum abgezogen. Das zurückbleibende Produkt wird bei 150°C unter UV-Bestrahlung chloriert. Die Reaktion ist nach Steigerung der Temperatur auf 190–200°C nach insgesamt etwa 5 Stunden beendet. Das zurückbleibende Produkt wird mit 5 g Antimon-pentachlorid versetzt. Bei 80–100°C wird Chlor bis zur Aufnahme der berechneten Menge (1 Mol-Äquivalent) eingeleitet. Man erhält 453 g Rohprodukt, $n_D^{20}$: 1,5825. Das Rohprodukt wird in einem Stahlautoklaven zu 500 ml wasserfreier Flusssäure gegeben und unter Rühren auf 140°C erhitzt. Der entstehende Chlorwasserstoff wird bei etwa 21 bar entspannt. Nach Beendigung der Gasentwicklung wird das Produkt im Vakuum destilliert.

Man erhält 300 g 3-Chlor-4-trifluormethoxybenzoylfluorid (vgl. Beispiel 17).

Beispiel c

$(CH_3)_3C{-}CO{-}F$

In einem metallischen Rührgefäss mit Kühler (V2A-Stahl) lässt man zu überschüssiger wasserfreier Flusssäure (500 ml) bei einer Temperatur von ca. -10°C 100 ml Pivaloylchlorid (vom Siedepunkt 103–104°C; $n_D^{20}$: 1,4168) zulaufen. Man lässt nach beendeter Zugabe die Temperatur bis auf ca. 15–18°C ansteigen und lässt bei dieser Temperatur ausreagieren. Wenn die Chlorwasserstoff-Entwicklung beendet ist (nach ca. 4 Stunden), wird durch Destillation aufgearbeitet.

Man erhält das Pivaloylfluorid als Flüssigkeit vom Siedepunkt 67°C und Brechungsindex $n_D^{20}$: 1,3558; Ausbeute ca. 80% der Theorie.

**Patentansprüche**

1) Verfahren zur Herstellung von Acylcyaniden der allgemeinen Formel

R–CO–CN (I),

in welcher
R für gegebenenfalls substituiertes Alkyl mit bis zu 8 Kohlenstoffatomen, gegebenenfalls substituiertes Cycloalkyl mit 3 bis 12 Kohlenstoffatomen, gegebenenfalls substituiertes Aryl oder für einen gegebenenfalls substituierten 5- oder 6-gliedrigen heterocyclischen Rest steht, der zusätzlich noch mit einem Benzolring anelliert sein kann,
durch Umsetzen von Carbonsäurefluoriden der allgemeinen Formel

R–CO–F (II),

in welcher R die oben angegebene Bedeutung hat, mit Alkalicyaniden, gegebenenfalls in Gegenwart eines Verdünnungsmittels, dadurch gekennzeichnet, dass die Umsetzung in Abwesenheit eines Katalysators, bei Temperaturen zwischen 10 und 200°C, durchgeführt wird.

2) Verfahren nach Anspruch 1, dadurch gekennzeichnet, dass man die Umsetzung beim Arbeiten in Gegenwart eines Verdünnungsmittels bei Temperaturen zwischen 20 und 150°C durchführt.

3) Verfahren nach Anspruch 1, dadurch gekennzeichnet, dass man die Umsetzung beim Arbeiten ohne Verdünnungsmittel bei Temperaturen zwischen 60 und 120°C durchführt.

4) Verfahren nach Anspruch 1, dadurch gekennzeichnet, dass man auf 1 Mol Carbonsäurefluorid (II) 0,7 bis 1,4 Mol Alkalicyanid einsetzt.

5) Verfahren nach Anspruch 1, dadurch gekennzeichnet, dass man auf 1 Mol Carbonsäurefluorid (II) 1 bis 1,25 Mol Alkalicyanid einsetzt.

6) Verfahren nach Anspruch 1, dadurch gekennzeichnet, dass man als Carbonsäurefluorid der Formel (II) Pivalinsäurefluorid, Benzoylfluorid oder 3-Trifluormethyl-benzoylfluorid einsetzt.

7) Verfahren nach Anspruch 1, dadurch gekennzeichnet, dass man als Alkalicyanid Natriumcyanid einsetzt.

**Claims**

1) Process for the preparation of acyl cyanides of the general formula

R-CO-CN (I),

in which
R represents optionally substituted alkyl with up to 8 carbon atoms, optionally substituted cycloalkyl with 3 to 12 carbon atoms, optionally substituted aryl or an optionally substituted 5-membered or 6-membered heterocyclic radical, which can additionally be fused to a benzene ring,
by reacting carboxyclic acid fluorides of the general formula

R-CO-F (II)

in which
R has the meaning indicated above, with alkali metal cyanides, optionally in the presence of a diluent, characterised in that the reaction is carried out without a catalyst, at temperatures between 10 and 200°C.

2) Process according to Claim 1, characterised in that when working in the presence of a diluent the reaction is carried out at temperatures between 20 and 150°C.

3) Process according to Claim 1, characterised in that when working without diluents the reaction is carried out at temperatures between 60 and 120°C.

4) Process according to Claim 1, characterised in that 0.7 to 1.4 mols of alkali metal cyanide are employed per mol of carboxylic acid fluoride (II).

5) Process according to Claim 1, characterised in that 1 to 1.25 mols of alkali metal cyanide are employed per mol of carboxylic acid fluoride (II).

6) Process according to Claim 1, characterised in that pivaloyl fluoride, benzoyl fluoride or 3-trifluoro-methyl-benzoyl fluoride is employed as the carboxylic acid fluoride of the formula (II).

7) Process according to Claim 1, characterised in that sodium cyanide is employed as the alkali metal cyanide.

**Revendications**

1) Procédé de préparation de cyanures d'acyle de formule générale

R-CO-CN (I)

dans laquelle
R représente un groupe alkyle éventuellement substitué contenant jusqu'à 8 atomes de carbone, un groupe cycloalkyle éventuellement substitué contenant 3 à 12 atomes de carbone, un groupe aryle éventuellement substitué ou un reste hétérocyclique à 5 ou 6 chaînons éventuellement substitué et qui peut en outre être condensé avec un noyau benzénique,
par réaction de fluorures d'acides carboxylique de formule générale

R-CO-F (II)

dans laquelle R a les significations indiquées ci-dessus, avec des cyanures alcalins, éventuellement en présence d'un diluant, caractérisé en ce que l'on effectue la réaction en l'absence de catalyseur, à des températures de 10 à 200°C.

2) Procédé selon la revendication 1, caractérisé en ce que, lorsqu'on opère en présence d'un diluant, on effectue la réaction à des températures de 20 à 150°C.

3) Procécé selon la revendication 1, cractérisé en ce que, lorsqu'on opère sans diluant, on effectue la réaction à des températures de 60 à 120°C.

4) Procédé selon la revendication 1, caractérisé en ce que l'on utilise de 0,7 à 1,4 mole de cyanure alcalin par mole de fluorure d'acide carboxylique II.

5) Procédé selon la revendication 1, caractérisé en ce que l'on utilise de 1 à 1,25 mole de cyanure alcalin par mole de fluorure d'acide carboxylique II.

6) Procédé selon la revendication 1, caractérisé en ce que l'on utilise en tant que fluorure d'acide carboxylique de formule II le fluorure de l'acide pivalique, le fluorure de benzoyle ou le fluorure de 3-trifluorométhylbenzoyle.

7) Procédé selon la revendication 1, caractérisé en ce que l'on utilise en tant que cyanure alcalin le cyanure de sodium.